(19) Europäisches Patentamt
European Patent Office
Office européen des brevets

(11) EP 1 343 012 A1

(12) EUROPÄISCHE PATENTANMELDUNG

(43) Veröffentlichungstag:
**10.09.2003 Patentblatt 2003/37**

(51) Int Cl.7: **G01N 33/58**, C12Q 1/26, G01N 33/543, G01N 33/552

(21) Anmeldenummer: **03004841.7**

(22) Anmeldetag: **05.03.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **08.03.2002 DE 10210224**

(71) Anmelder: **Infineon Technologies AG**
**81669 München (DE)**

(72) Erfinder:
- **Hanke, Hans-Christian, Dr.rer.nat.**
  **81479 München (DE)**
- **Martin, Alfred, Dipl.-Phys.**
  **93053 Regensburg (DE)**

(74) Vertreter: **Müller-Boré & Partner Patentanwälte**
**Grafinger Strasse 2**
**81671 München (DE)**

(54) **Nachweis von Analyten mit Signalverstärkung durch Polymerisation**

(57) Die vorliegende Erfindung betrifft ein hochempfindliches Analysesystem zum Nachweis von Analyten, insbesondere von biologischem Material, in einer Probe, worin ein positives Signal durch *in-situ*-Erzeugung eines Polymerisats amplifiziert wird. Ferner betrifft die vorliegende Erfindung einen, dieses Analysesystem enthaltenden Kit sowie ein Verfahren zum Nachweis von auf einem Träger immobilisierten Analyten unter Verwendung dieses Analysesystems.

EP 1 343 012 A1

## Beschreibung

Die vorliegende Erfindung betrifft ein hochempfindliches Analysesystem zum Nachweis von Analyten, insbesondere von biologischem Material, in einer Probe, worin ein positives Signal durch *in situ*-Erzeugung eines Polymerisats amplifiziert wird. Ferner betrifft die vorliegende Erfindung einen, dieses Analysesystem enthaltenden Kit sowie ein Verfahren zum Nachweis von auf einem Träger immobilisierten Analyten unter Verwendung dieses Analysesystems.

Der Nachweis von Biomolekülen ist für viele Anwendungen in der forschenden Pharmaindustrie und in der klinischen Diagnostik von zentraler Bedeutung. In der Regel erfolgt dieser Nachweis über eine sogenannte Affinitätsreaktion, bei der komplementäre Analyten sich gegenseitig spezifisch binden. Grundsätzlich besteht das Problem, daß der nachzuweisende Analyt nur in geringsten Konzentrationen in einem Testansatz zur Verfügung steht. Meist ist die Sensitivität heutiger Meßsysteme zu gering, um diese niedrigen Kozentrationen eines Analyten direkt nachzuweisen. Man verwendet daher Amplifikationssysteme, die das biologische Signal derart indirekt verstärken, daß die untere Nachweisgrenze der Meßapparatur möglichst weit überschritten wird.

Affinitätssensoren sind spezielle Biosensoren, die jede Art biomolekularer Erkennung von hochspezifischen Affinitätspartnern nutzen, wie z.B. Antikörper-Antigen, Nukleinsäure-komplementäre Nukleinsäure oder Rezeptor-Ligand. Bio- und Affinitätssensoren setzen sich im allgemeinen aus zwei Komponenten zusammen: der biologischen Komponente, zur spezifischen Erkennung eines Analyten, und der Detektorkomponente, dem Transducer (i.e. Signal bzw. Meßwandler), der diese biomolekulare Erkennungsreaktion erfassen und in ein auswertbares Signal umsetzen soll. Die selektiven biologischen Komponenten (z.B. Nukleinsäuren, Enzyme, Antikörper, Antigene oder Mikroorganismen) sind in direkter räumlicher Nähe zum Transducer immobilisiert und lassen sich heute durch ein breites Spektrum an Meßprinzipien nachweisen. Die Wahl des Transducers richtet sich nach der Reaktion der biologischen Komponente und den daraus resultierenden Änderungen. Im Stand der Technik sind eine Reihe von Transducern beschrieben, die nach unterschiedlichen Prinzipien arbeiten. Gebräuchlich sind Transducer auf elektrochemischer, elektrischer oder optischer Basis. Massensensitive Meßwandler werden zur Detektion von Bindungen auf Oberflächen oder Antikörpern verwendet. Je mehr Analyt gebunden wird, umso kleiner wird die Schwingungsfrequenz des verwendeten Quarzträgers.

Sensitivität und Spezifität der biomolekularen Erkennung kennzeichnen - neben anderen Parametern wie Reproduzierbarkeit, Drift, Herstellungskosten, Handhabbarkeit - die Qualität und Praxistauglichkeit der Sensoren. Während die Spezifität des Biosensors weitgehend durch die biologische Komponente vorgegeben ist, wird seine Empfindlichkeit vor allem durch den verwendeten Transducer bestimmt. Es ist daher wünschenswert, für eine gegebene biologische Nachweisreaktion einen möglichst sensitiven Transducer zu verwenden.

Grundsätzlich unterscheidet man zwischen markierungsfreien Verfahren, die Biomoleküle direkt nachweisen können, und Verfahren, bei denen der Analyt markiert wird, um ihn mit Hilfe dieses Markers zu detektieren.

Primär Markierungsfreie Verfahren haben in der Regel einen der beiden Bindungspartner an ihrer Oberfläche gebunden, um damit spezifisch den anderen Partner zu detektieren. Aufgrund der direkten Detektion (Immunosensor) und der fehlenden Beeinflussung der Affinität durch Marker werden solche Verfahren oft zur Charakterisierung kinetischer oder thermodynamischer Parameter der Wechselwirkung eingesetzt. Vor allem optische Verfahren haben sich etabliert, wie z.B. die Oberflächenplasmonenresonanz (SPR), der Prismenkoppler (Resonant Mirror) und Gitterkoppler, die auf einer Wechselwirkung mit dem evaneszenten Feld beruhen und Änderungen des Brechungsindex nahe der Sensoroberfläche detektieren.

Neben optischen Verfahren hat auch die akustische Detektion, mit welchen die Frequenzänderungen eines piezoelektrischen Schwingquarzes durch Masseänderung hochempfindlich nachgewiesen werden kann, Verbreitung gefunden. Hier kommen QCM (Quartz crystal microbalance)- und SAW (Surface acoustic wave) - Methoden zum Einsatz.

Verfahren mit markierten Komponenten werden aufgrund ihrer meist geringeren Nachweisgrenzen häufig zur Analytik vor allem auch in der Routinediagnostik herangezogen. Hierbei trägt mindestens eine der definiert zugegebenen Komponenten der affinen Bindungspartner einen Marker, der im Anschluß eine quantitative bzw. qualitative Bestimmung ermöglicht. Zum einen kann die Markierung eine (chemische) Verbindung oder Gruppe sein, die unmittelbar in der Lage ist, ein zur Erfassung der makromolekularen Biomoleküle einsetzbares Signal zu erzeugen. Die Erzeugung dieses Signals kann dabei extern angeregt werden, jedoch kann die Markierung das Signal auch ohne äußere Anregung abgeben. Im erstgenannten Fall ist die Markierung beispielsweise ein Fluoreszenz-Farbstoff (Fluorophor) oder ein Chemilumineszens-Farbstoff, im zweitgenannten Fall beispielsweise ein Radioisotop. Zum anderen kann die Markierung eine Substanz sein, die nur mittelbar ein Signal zur Erfassung der makromolekularen Biomoleküle erzeugt, d.h. eine Substanz, welche die Erzeugung des Signals veranlasst. Beispielsweise kann eine solche Reportergruppe ein Enzym sein, welches eine chemische Reaktion katalysiert, und die chemische Reaktion zur Erfassung der Biomoleküle herangezogen wird.

Beispiele für solche Enzyme sind Alkalische Phosphatase, Glutathion-S-Transferase, Superoxid-Dismutase,

Meerrettich-Peroxidase, α-Galaktosidase und β-Galaktosidase. Diese Enzyme sind in der Lage, geeignete Substrate zu spalten, die z. B. farbige Endprodukte ergeben. Zu der Gruppe der Markierungen, die nur mittelbar ein zum Nachweis von makromolekularen Biomoleküle einsetzbares Signal erzeugen, gehören ferner Liganden für Bindungsproteine und Substrate für Enzyme. Diese Markierungen werden hier allgemein als Enzym-Liganden bezeichnet. Beispiele für solche als Markierung einsetzbare Enzym-Liganden sind Biotin, Digoxigenin oder Substrate für die oben genannten Enzyme.

Ein typischer Vertreter eines derartigen Testsystems, das in diese Klasse fällt ist der sogenannte ELISA (Enzyme Linked Immuno Sorbent Assay). Hier ist der sogenannte "Sandwich-Assay" am gebräulichsten. Das Verfahren verwendet üblicherweise ein gebundenes Antigen, das von einem ersten, spezifischen, gegen dieses Antigen gerichteten Primärantikörper gebunden wird. Der Nachweis erfolgt dann durch einen Sekundärantikörper, der spezifisch an den Primärantikörper bindet ("Sandwichaufbau"). Wenn der Sekundärantikörper beispielsweise mit alkalischer Phosphatase konjugiert ist, so erfolgt der letztendliche Nachweis auf diesem indirekten Weg über ein durch dieses Enzym umsetzbares Farbsubstrat.

Radioaktive Verfahren gehören zu den empfindlichsten immunologischen Testformaten mit einer Nachweisgrenze (nach Vorbehandlung der Probe) von bis zu 10 fmol/L. Ein klassischer Vertreter ist der "Radio-Immunoassay" (RIA).

Amplifikationssysteme für Affinitätssensoren werden sowohl für primär markierungsfreie als auch für Verfahren mit markierten Komponenten verwendet. Unter primär markierungsfrei wird hier verstanden, daß der entsprechende Transducer prinzipiell in der Lage ist, beispielsweise Biomoleküle ohne Markierung zu detektieren. Dennoch verwendet man auch bei diesen Verfahren markierte Komponenten um die Sensitivität des Meßsystems zu erhöhen.

Die Limitierung als ein Nachteil dieser Verfahren liegt unter anderen darin, daß diese Signalamplifikation nur linear skaliert, d.h. das Signal ist linear abhängig von der Anzahl der signalerzeugenden Moleküle oder dem Substratumsatz des Reporterenzyms.

Somit liegt der vorliegenden Erfindung die Aufgabe zugrunde, ein neues System und Verfahren bereitzustellen, welche eine verbesserte Signalamplifikation von biologischen Bindungsreaktionen, wie Antigen-Antikörper-Reaktionen oder Nukleinsäure-Hybritisierungen, erzeugen sollen und vorzugsweise die Nachweisgrenze herabsetzen sollen.

Diese Aufgabe wird durch die in den Ansprüchen gekennzeichneten Ausführungsformen der vorliegenden Erfindung gelöst. Insbesondere wird ein Analysesystem bereitgestellt, enthaltend ein Detektormolekül, welches zur spezifischen Bindung an den zu bestimmenden Analyten befähigt ist, und ein Katalysatorsystem, welches an die Detektormoleküle gebunden vorliegt oder zur spezifischen Bindung an die Detektormoleküle befähigt ist, wobei das Katalysatorsystem (i) zur katalytischen Herstellung eines Polymerisats oder (ii)zur katalytischen Erzeugung eines Polymerisationsinitiators oder einer Vorstufe davon für die Herstellung eines Polymerisats befähigt ist. Das erfindungsgemäße Analysesystem umfaßt somit die "biologische Komponente" zur spezifischen Erkennung eines Analyten und den "Transducer".

In einer Ausführungsform der vorliegenden Erfindung liegen die Detektormoleküle und das Katalysatorsystem zunächst getrennt, d.h. nicht kovalent gebunden, im Analysesystem vor, worin ein Detektormolekül mindestens einen Analyt-bindenden Bereich und mindestens einen Katalysatorsystem-bindenden Bereich umfaßt und das Katalysatorsystem mindestens einen Detektormolekül-bindenden Bereich umfaßt. Diese Bereiche sind vorzugsweise und unabhängig voneinander aus der Gruppe, bestehend aus Antigen-bindenden Antikörperstrukturen, Haptenstrukturen, Nukleinsäuresequenzen oder komplementäre Sequenzen/Strukturen-bindenden Derivaten davon, Ligandensystemen, wie Rezeptor-Ligand-Systemen und Polypeptid-affinen-Systemen, wie beispielsweise Biotin-Avidin-Affinität, ausgewählt. In einer bevorzugten Ausführungsform der vorliegenden Erfindung ist das Detektormolekül ein Antikörper oder eine Nukleinsäuresequenz, die ein oder mehrere daran konjugierte Biotinmoleküle, enthalten, wobei hier der Detektor-bindende Bereich des Katalysatorsystems mit vorzugsweise Avidin bzw. Streptavidin umfaßt.

In einer anderen Ausführungsform der vorliegenden Erfindung betrifft das Analysesystem ein Detektormolekül mit mindestens einem vorstehend definierten Analyt-bindenden Bereich, wobei das Detektormolekül mindestens ein Katalysatorsystem kovalent gebunden enthält. Das Katalysatorsystem kann gegebenenfalls über einen Spacer, wie beispielsweise Oligopeptide und Derviate davon oder bifunktionelle Verbindungen, wie Maleinsäurederivate mit dem Detektormolekül verknüpft sein.

In einer bevorzugten Ausführungsform der vorliegenden Erfindung enthält der Bereich des Katalysatorsystems zur katalytischen Erzeugung eines Polymerisationsinitiators oder einer Vorstufe davon mindestens ein Enzym aus Gruppe der Oxidoreduktasen bzw. Oxidasen, beispielsweise Lactatoxidase, Galactoseoxidase, L-2-Hydroxysäureoxidase, Glucoseoxidase, Glycolatoxidase, Hexoseoxidase, L-Gulonolactonoxidase, L-Sorboseoxidase, Pyridoxol-4-oxidase oder Alkoholoxidase.

Der Polymerisationsinitiator kann ein radikalischer oder ein ionischer (anionischer oder kationischer) Polymerisationsinitiator sein.

Das erfindungsgemäß herzustellende Polymerisat ist aus polymerisierbaren Verbindungen, wie Monomere oder Oligomere, aufgebaut und kann durch Lösungs-, Emulsions- oder Suspensionspolymerisation erhalten werden.

Beispiele für polymerisierbare Verbindungen sind α,β-ungesättigte Monooder Dicarbonsäuren wie Maleinsäure, Fumarsäure, Acrylsäure, Methacrylsäure, Itaconsäure, α,β-ungesättigte Carbonäureamide wie Maleindiamed, Maleinsäuremonoamide, Fumarsäurediamid, Acrylsäure, Methacrylsäureamid, Itaconsäureamid, Hydroxylgruppen-haltige Monomere mit mindestens einer polymerisierbaren ethylenisch ungesättigten Gruppe wie (Meth)acrylsäure-hydroxylalkylester, beispielsweise β-Hydroxyethylacrylat, β-Hydroxypropylmeth-acrylat, 1,4-Butandiol-4-monoacrylat, Propylenglycolmonoacrylat, 2,3-Dihydropropylmethacrylat, Pentaerythritmonomethacrylat, Polypropylenglycolmonoacrylat, Fumarsäuredihydroxyalkylester, n-Hydroxyalkyl(meth)acrylamide, n-Hydroxyalkylfumarsäuremonooder -diamide, beispielsweise n-Hydroxyethyl-Alkylamid oder n-(2-Hydroxypropyl)methacrylamid, und Aminogruppen-haltige Monomere mit mindestens einer polymerisierbaren ethylenisch ungesättigten Gruppe wie n-Dialkyl- oder n-Monoalkylaminoalkyl (meth)acrylate, beispielsweise n-Diethylaminoethylmethacrylat, n-tert-Butylaminoethylacrylat oder die entsprechenden n-Alkanolverbindungen, N-Dialkyloder N-Monoalkylaminoalkyl(meth)acrylamid, beispielsweise N-Dimethylaminoethylacrylamid oder die entsprechenden N-Alkanolverbindungen oder Vinylgruppen enthaltende heterocyclische Verbindungen mit einem oder mehreren basischen Stickstoffatomen wie beispielsweise n-Vinylimidazol. Weitere Beispiele für erfindungsgemäß einsetzbare Monomere sind Acrylsäurealkylester, Methacrylsäurealkylester, Maleinsäuredialkylester, Fumarsäuredialkylester, wobei die Alkylreste vorzugsweise 1 bis 20 Kohlenstoffatome aufweisen und in linearer oder verzweigter aliphatischer Kette und/oder als cycloaliphatischer und/oder (Alkyl)-aromatischer Rest angeordnet sind. Spezifische Beispiele hierfür sind Styrol und Derivate davon, Methylmethacrylat, Ethylmethacrylat, Butylmethacrylat, Cyclohexylmethacrylat, Isobornylmethacrylat, Dihydrodicyclopentadienylmethacrylat, (Meth)acrylamid, (Meth)acrylnitril, n-Butylacrylat, Isobutylacrylat, tert-Butylacrylat, 2-Ethylhexylacrylat, Laurylacrylat, Ethoxyethylmethacrylat und Tetrahydrofurfurylacrylat.

Das Polymerisat kann ein Homo- oder Copolymerisat, abhängig von den eingesetzten Monomeren, sein.

Der mit dem erfindungsgemäßen Analysesystem zu bestimmende Analyt kann eine niedermolekulare organische Verbindung wie Pestizide, Herbizide, Fungizide,Drogen (gemeint sind Drugs of Abuse), Konservierungsstoffe, Medikamente oder ein biologisches Material sein. Beispiele für das biologische Material sind Polypeptide oder Fragmente und Derivate davon, einzel- oder doppelsträngig vorliegende Nukleinsäuren oder Fragmente und Derivate davon, Lipide, Hormone, Stoffwechselprodukte, Zuckerverbindungen, eukaryontische und prokaryontische Zellen oder Zellbestandteile davon, Antigen-Antikörper-Komplexe sowie Rezeptor-Liganden.

Besonders bevorzugte Ausführungsformen des erfindungsgemäßen Analysesystems sind (i) ein mit einem Biotinmolekül konjugierter Antikörper als Detektormolekül und ein Katalysatorsystem, welches ein Avidinmolekül und daran, vorzugsweise über einen Spacer gekoppeltes Enzym wie die Glucoseoxidase (GOD) enthält (vgl. Fig. 1A), (ii) ein Antikörper und ein daran kovalent, vorzugsweise über einen Spacer gebundenes Enzym wie Glucoseoxidase (vgl. Fig. 1B).

Ein weiterer Gegenstand der Erfindung betrifft einen Kit, welcher das vorstehende Analysesystem enthält.

Ein weiterer Gegenstand der Erfindung ist ein Verfahren zum Nachweis von auf einem Träger immobilisierten Analyten, umfassend die Schritte:

(a) Inkontaktbringen des immobilisierten Analyten mit dem vorstehend definierten Analysesystem unter Ausbildung eines Analyt-Analysesystem-Komplexes,

(b) Starten einer Polymerisation

(i) nach Zugabe der vorstehend polymerisierbaren Verbindungen oder
(ii) nach Zugabe eines Substrats zur katalytischen Erzeugung eines Polymerisationsinitiators oder einer Vorstufe davon und der polymerisierbaren Verbindungen

unter Herstellung eines vorstehend definierten Polymerisats und

(c) Auswerten der Polymerisation unter Bestimmung des Polymerisats.

Das in Schritt (a) durchgeführte Inkontaktbringen ist vorzugsweise eine Antigen-Antikörper-Reaktion oder eine Hybridisierung.

Die in Schritt (b) des erfindungsgemäßen Verfahrens durchgeführte Polymerisation ist vorzugsweise eine radikalische Polymerisation.

Die Initiierung der radikalischen Polymerisation erfolgt häufig durch Verwendung von verschiedenen Peroxid-Derivaten.

Zur Beschleunigung der Startreaktionen werden die Peroxide in Kombination mit geeigneten Reduktionsmitteln, wie Metallsalze oder Aminen eingesetzt. Im Stand der Technik sind Verfahren zur Polymerisation oder von radikalisch polymerisierbaren, in wäßrigen Systemen gelösten oder dispergierten Monomeren unter Verwendung eines

Radikalinitiators beschrieben.

Nachstehend wird beschrieben wie der durch das erfindungsgemäße Verfahren bereitgestellte Mechanismus für eine Signalamplifikation bei Affinitätssensoren zu nutzen ist, sodaß das erfindungsgemäße Verfahren als Signalamplifikation mit einer Vielzahl von herkömmlichen "Transducern" ohne Modifikation verwendet werden kann.

Das erfindungsgemäße Verfahren verwendet in einer bevorzugten Ausführungsform ein Enzym aus der Gruppe der Oxidasen bzw. Oxidoreduktasen, wie vorstehend definiert, ein Substrat dieses Enzyms, einen Radikalinitiator und molekularen Sauerstoff als Cosubstrat sowie unterschiedliche polymerisierbare Verbindungen, die radikalisch kondensiert werden können.

Ein besonders bevorzugtes Enzym ist die Glucoseoxidase (GOD). Sie katalysiert unter Verwendung von z. B. molekularen Sauerstoff als natürlichem Cosubstrat die Oxidation von $\alpha$-D-Glucose zu D-Glucono-$\gamma$-lacton unter Erhalt von $H_2O_2$:

$$\text{ß-D-Glucose} + O_2 \xrightarrow{\text{GOD(FAD)}} \text{D-Glucono-}\gamma\text{-lacton} + H_2O_2$$

$$\text{D-Glucono-}\gamma\text{-lacton} \xrightarrow{\text{Hydrolyse}} \text{Gluconsäure}$$

Durch die sogannte Fenton-Reaktion erhält man in Gegenwart von $Fe^{2+}$ und leicht saurem pH ein hochreaktives Hydroxyradikal:

$$Fe^{2+} + H_2O_2 \rightarrow Fe^{3+} + OH^{\cdot} + OH^{-}$$

Auch TEMED (N,N,N'N'-Tetramethylendiamin) sowie wasserlösliche Metallsalze in ihren niedrigen Wertigkeitsstufen, wie Kobalt-(II)- oder Kuper-(I)-Salze. z.B. Eisen-II-chlorid, führen zu einer - wie oben beschriebenen - Reduktion des Peroxids unter Bildung von einem Hydroxylradikal.

Als polymerisierbare Verbindungen kommen praktisch alle radikalisch polymerisierbaren Monomeren in Frage, wobei jedoch die üblichen Einschränkungen für Copolymerisationen gelten, die durch das Q-und e-Schema nach Alrey und Price bzw. die Copolymerisations parameter vorgegeben sind (vgl. z.B. Brandrup und Immergut, Polymer Handbook. 2nd Edition, John Wiley and Sons. New York. 1975). Beispiele für polymerisierbare Verbindungen sind vorstehend aufgeführt.

Bevorzugt werden solche (ungesättigten) Verbindungen, z.B. Acrylverbindungen, eingesetzt, deren Löslichkeit in Wasser mindestens 0,1 pro 100 g Wasser, bevorzugt mindestens 1 g pro 100 g Wasser allein oder im Gemisch beträgt.

In einer bevorzugten Ausführungsform des erfindungsgemäßen Verfahrens ist der Träger bzw. das Substrat aus einem gegebenenfalls porösen Halbleitermaterial aufgebaut. Das (poröse) Halbleitermaterial ist vorzugsweise aus Silizium, Siliziumoxid oder Silizium mit mindestens einer oder mehreren (unterschiedlichen) Schichten, ausgewählt aus Siliziumoxid, Siliziumnitrid, Germanium und Galliumarsenid, ausgewählt. Besonders bevorzugt ist ein poröser Silizium-Chip mit Poren bzw. Kanälen, welche sich von einer Oberfläche des Chips zur gegenüberliegenden Oberfläche erstrecken (sog. poröser Chip, oder engl. "flow-through chip", FTC). Die Poren bzw. diskrete Kanäle des Halbleitermaterials weisen beispielsweise einen Durchmesser von etwa 500 nm bis etwa 50 $\mu$m auf. Die Dicke des Trägermaterials liegt beispielsweise im Bereich von 400 $\mu$m bis 700 $\mu$m, vorzugsweise etwa 500 $\mu$m.

In Schritt (c) des erfindungsgemäßen Verfahrens wird die Auswertung der Polymerisation vorzugsweise durch optische, elektrische und/oder gravimetrische Bestimmung des Polymerisats durchgeführt. Das erfindüngsgemäße Verfahren ist prinzipiell auf alle bekannten Transducer-Prinzipien anwendbar. Diese Bestimmungsmethoden werden nachfolgend beispielhaft näher erläutert:

1. Optische Detektion

Hier wird ausgenutzt, dass sich durch die Polymerisation lokal die optischen Eigenschaften des Substrats ändert.

(a) Transmissionsmessung/Absorptionsmessung

Durch das Einlagern von Polymeren in das makroporöse Si werden die optischen Eigenschaften verändert.

Die Transmission des Polymers kann durch die Synthese der Monomere gesteuert werden. Es ist möglich die Transmission in dem gewünschten Wellelängenbereich zu minimieren. Ein prinzipieller Aufbau zur Detektion ist wie folgt:

Methode I: Der lokal ("Dots") mit dem Polymeren gefüllte Biochip, d.h. der Träger nach Schritt (b) des erfindungsgemäßen Verfahrens, wird von oben mit einer Lichtquelle homogen beleuchtet und das transmittierte Licht von unten mit einer CCD Kamera aufgenommen. Die Wellenlänge liegt zwischen 1,6 µm und 250 nm, vorzugsweise aber im sichtbaren Spektrum.

Dort, wo die Analyt-Analysesystem-Bindung, beispielsweise eine Hybridisierung, stattgefunden hat und sich ein Polymer gebildet hat, ist die Transmission durch die zusätzlich in die Poren eingebrachte Materie verringert. Die lokal verringerte Transmission äußert sich in einem Helligkeitsunterschied. Der detektierte Intensitätsunterschied ist ein Maß für die lokale Analyt-Analysesystem-Komplex-Konzentration, beispielsweise der Hybridisierungsdichte. Bei der Wahl der Monomeren ist zu berücksichtigen, dass die Absorption im gewünschten Wellenlängenbereich liegt und sich vom Absorptionsspektrum des Substrates unterscheidet. Das Absorptionsspektrum des Polymers wird durch die chemische Zusammensetzung der Monomere bestimmt. Es können auch Farbstoffmoleküle in die Polymermatrix bzw. Monomere eingebracht und das Polymer dadurch eingefärbt werden. Als kompakte Lichtquelle kann eine planare LED oder eine OLED Schicht verwendet werden (vgl. Fig. 5A).

Methode II: Der poröse Si-Biochip nach Schritt (b) des erfindungsgemäßen Verfahrens befindet sich zwischen einer LED Matrix und einem Photodetektor. Die LED-Matrix beleuchtet nacheinander jeden Dot und es wird simultan der Strom in dem Detektor gemessen. Man erhält für jeden Dot einen bestimmten Sensorstrom, der je nach erfolgter Polymerisation niedriger oder höher ist, da die Transmission verringert wurde. Es wird ein sehr kompakter Sensor erhalten (vgl. Fig. 5B).

(b) Detektion von Streulicht auf planarem Substrat bzw. Träger

Die ortsaufgelöste Detektion von Streulicht zur Messung von Parkikelverteilungen auf einer Waferoberfläche ist ein weit verbreitetes Verfahren (KLA Tencor SP1, Darkfieldinspection etc.) in der Halbleiterfertigung. Dieses Verfahren kann auch zur Auswertung von beispielsweise hochparallelen DNA Arrays verwendet werden. Auf einen Träger werden lokal unterschiedliche Fängermoleküle immobilisiert. Anschließend wird der Analyt aufgebracht. Es bildet sich nach dem oben beschriebenen Verfahren ein Polymer lokal an den Stellen, an denen eine Hybridisierung stattgefunden hat. Es verändern sich also lokal die Eigenschaften der Oberfläche, insbesondere die Topologie der Oberfläche. Geht man von einer Dotgröße von 100 µm aus, dann bildet sich ein 100 µm großer (in der Größenordnung µm dicker) "Polymerpfropf", der mit konventionellen Laserscannern, wie sie aus der Halbleiterindustrie bekannt sind, detektiert werden kann.

Beim Auslesen des Arrays rotiert der Wafer/Substrat bzw. Träger und es wird ortsaufgelöst das Streulicht über einen ortsfesten Detektor detektiert. Alternativ kann auch das Substrat in x-y bewegt werden.

Alternativ kann statt des Si-Substrates auch ein strukturierter Kunststoffträger verwendet werden. Der Kunststoffträger für die biologischen Experimente kann einfach durch eine Prägetechnik hergestellt werden.

Die Diffusion des zur Polymerisation notwendigen $H_2O_2$ begrenzt die laterale Auflösung und es kann vorkommen, dass es zu einem Überlappen (Übersprechen) verschiedener "Dots" kommt. Eine Möglichkeit besteht darin, in das Substrat kleine den Dotgrößen entsprechende Vertiefungen zu ätzen, die dazu dienen, die Polymerisation nur in einem sehr begrenzten Volumen starten zu lassen und ein "Übersprechen" durch den Einschluss zu verhindern. Die Vertiefungen werden bei der Polymerisation aufgefüllt und haben im Vergleich zum Substrat unterschiedliche optische Eigenschaften (Brechungsindex, Absorptionseigenschaften, Reflektivität etc.) (vgl. Fig. 6).

(c) Fluoreszenzmessung

Da viele Polymere Fluoreszenz Eigenschaften besitzen, kann die "polymerverstärkte" Detektion von Biomolekülen auch mit konventionellen Fluoreszenzscannern ausgewertet werden. Die Fluoreszenz Eigenschaften und die spektrale Verteilung der Fluoreszenz können über die Zusammensetzung der Monomere beeinflusst werden.

2. Elektrische Detektion

Hier wird ausgenutzt, dass sich durch die Polymerisation lokal die elektrischen Eigenschaften des Substrats bzw. Trägers oder einer Sensoranordnung ändert.

(a) kapazitive Änderung/Impedanzauswertung

Die kapazitive Auswertung kann sowohl bei planaren Substraten als auch bei porösen Substraten stattfinden.

Bei planaren Substraten werden Interdigital Strukturen (ITD) als Sensorelektroden aufgebracht. Beispielsweise wird ein Hybridisierungsereignis durch die Polymerisation verstärkt, und es bildet sich lokal eine Polymerschicht, welche die Interdigital Struktur bedeckt oder überdeckt. Dies führt zu einer veränderten Kopplung (Impedanz) zwischen den Elektrodenpaaren und zu einem elektrisch auswertbaren Signal. Das Prinzip ist bekannt (vgl. Fig. 7A).

Durch das lokale Einlagern von Polymeren in poröse Substrate bzw. Träger, wie beispielsweise das makroporöse Si, werden die elektrischen Eigenschaften verändert. Falls das Polymer eine unterschiedliche Dielektrizitätskonstante (DK) im Vergleich zu Luft hat, so kann die durch die Polymerisation verursachte Veränderung der DK detektiert werden. Durch geschickte Wahl der Monomere kann die Dielektrizitätskonstante des Polymers optimiert werden.

Auf den porösen Si-Chip werden auf die Vorderseite und der Rückseite in einer Arrayform Elektrodenflächen aus Gold aufgebracht, wobei die Sensoroberfläche etwa 100 µm Durchmesser betragen soll. Die Sensorflächen von Vorder- und Rückseite sollen zueinander ausgerichtet sein, so dass sich die Elektroden gegenüberliegen und mit dem dazwischenliegenden porösen Silizium einen Kondensator bilden. Die Elektrodenflächen haben den Durchmesser der Dots.

Anschließend wird der Chip wie oben beschrieben zur Detektion der Hybridisierung benutzt. Die vorzugsweise mit $SiO_2$ beschichteten Poreninnenwände sind vorzugsweise mit Fängermolekülen bedeckt. Auf den Metallelektroden haften die Fängermoleküle nicht. Die lokal durch die Hybridisierung ausgelöste Polymerisierung auf dem Chip führt dazu, dass sich lokal die Dielektrizitätskonstante ändert. Im Idealfall sind die Poren mit Polymeren ausgefüllt (vgl. Fig. 7B).

Im einfachsten Fall wird zur Messung an den beiden gegenüberliegenden Elektrodenflächen eine Wechselspannung angelegt und die Kapazität bestimmt. Das Hybridisierungsereignis wird durch eine Veränderung der Kapazität, das der Dot mit den Elektrodenflächen bildet, detektiert.

In einer weiteren Ausführungsform können die Elektrodenflächen vom Substrat getrennt ausgeführt werden, d.h. die Elektrodenflächen sind über einen Luftspalt an das poröse Si gekoppelt.

Durch entsprechende Wahl der Elektrodengeometrien (wie Ringleiter, Interdigital Strukturen, einfache Plattenanordnung) kann die kapazitive Kopplung zwischen den Dots verringert werden.

(b) Messung des elektrischen Widerstandes

Durch das lokale Einlagern von elektrisch leitfähigen Polymeren in poröse Substrate, wie beispielsweise das makroporöse Si, werden die elektrischen Eigenschaften verändert. Die durch die Hybridisierung gestartete Polymerisation kann einfach durch eine Widerstandsmessung ausgewertet werden.

Auf einem porösen Si-Chip werden auf der Vorderseite und der Rückseite in einer Arrayform Elektrodenflächen z.B. aus Gold aufgebracht, wobei die Sensorfläche etwa 100 µm Durchmesser betragen soll. Die Sensorflächen von Vorder- und Rückseite sollen zueinander ausgerichtet sein, so dass sich die Elektroden gegenüberliegen. Die Elektrodenflächen haben den Durchmesser der Dots. Wenn die elektrisch leitfähigen Polymere die Pore ausfüllen, so ändert sich die Leitfähigkeit zwischen den beiden Elektroden. Die Messung im nassen Zustand ist nicht einfach möglich, da die Ionenleitfähigkeit der mit Flüssigkeit gefüllten Pore einen "Untergrund" zu der Leitfähigkeit der Polymere darstellt. Die Messung des Widerstandes soll vorzugsweise im trockenen Zustand ausgeführt werden. Es kann aber auch die veränderte Ionenleitfähigkeit im Elektrolyten aufgrund des verringerten Porendurchmessers zur Detektion benutzt werden.

Bei planaren Substraten bzw. Trägern wird die resistive Kopplung zwischen zwei Elektroden ausgewertet. Die beispielsweise durch ein Hybridisierungsereignis ausgelöste lokale Polymerisation erzeugt eine Polymerschicht zwischen den Elektroden, wobei das Polymer den Widerstand zwischen den Elektroden ändert. Der gemessene Widerstand unterscheidet sich je nach dem, ob sich ein Polymer gebildet hat oder nicht. Es können sowohl Interdigital Strukturen als auch einfache Leiterbahnen verwendet werden.

2. gravimetrische Sensoren

Die durch beispielsweise eine die Hybridisierung gestartete Polymerisation verursacht eine Massenerhöhung, d.h. ein Hybridisierungsereignis wird in eine messbare Massenänderung umgewandelt.

Als mögliche Sensorprinzipien kommen in Frage:

- akustoelektrischer Effekt (Piezoschwinger (QCM based Systems), SAW, BAW);
- Cantilever (AFM)

Nachfolgend werden besondere Vorteile des erfindungsgemäßen Anlagesystems bzw. Verfahrens angeführt:

- Das erfindungsgemäße Analysesystem erzeugt durch eine beispielsweise enzymatisch katalysierte Reaktion freie

Radikale, die durch eine Kettenreaktion monomere Moleküle im Reaktionsgemisch zu einem Polymer kondensieren. Ist die Kettenreaktion erst einmal gestartet, erfolgt die weitere Polymerisation unabhängig von der Neubildung weiterer Radikale. Durch die Entkopplung der enzymatischen Neubildung dieser Radikale und dem radikalisch initiierten Polymerwachstum erreicht man eine sehr effiziente Signalverstärkung. Gegenüber herkömmlichen Systemen, die biologische Signale auf andere Weise nur linear verstärken, wird durch diesen entkoppelten Zweifachmechanismus eine potenzierte Signalverstärkung erreicht. Ein Enzym aus der Gruppe der Oxidoreduktasen katalysiert die fortdauernde Neubildung von Hydroxylradikalen, die wiederum die beschriebene Kettenreaktion mittelbar fortsetzen.

- Da das Polymer in wässriger Lösung entsteht, ergibt sich vor allem bei dessen Quervernetzung durch den Wassereinschluß eine weitere nicht-lineare Amplifikation des Signals, wobei die Eigenschaften des Polymers über den Quervernetzungsgrad in einfacher Weise gesteuert werden können.
- Eine weitere leichte Einflußmöglichkeit auf das enstehende Produkt ergibt sich dadurch, daß man die stöchometrische Variation der Reaktionsedukte, i.e. insbesondere Substrat-, und Monomerkonzentration, in geeigneter Weise variiert und auf die entsprechende biologische Nachweisreaktion, den Transducer oder das Meßverfahren abstimmt.
- Eine Positivreaktion (Polymerbildung) läßt sich sehr deutlich von der Negativreaktion (keine Polymerbildung) unterscheiden, wobei ein besonders vorteilhafter Signal/Rauschabstand gewährleistet ist.
- Das Verfahren läßt sich durch seine physiko-chemischen Eigenschaften ohne Modifikation für die verschiedensten Transducersysteme verwenden, da sich sowohl die Änderung der Masse, die Änderung der Absorption bzw. Reflektion sowie die Änderung der elektrischen Eigenschaften (Impedanz, Kapazität, Leitfähigkeit) messen lassen.
- Da das Polymer *in situ* gebildet wird, läßt sich seine Entstehung durch die oben beschriebenen Systeme leicht erfassen, wobei kinetische Untersuchungen ermöglicht werden.

Die Figuren zeigen:

Figur 1 ist eine schematische Darstellung von zwei bevorzugten Ausführungsformen des erfindungsgemäßen Analysesystems.

Figuren 2 bis 4 sind schematische Darstellungen von drei bevorzugten Ausführungsformen des erfindungsgemäßen Verfahrens.

Figur 5 zeigt in (A) und (B) schematisch die optische Detektion von Polymeren mittels Transmissions/Absorptionsmessungen.

Figur 6 zeigt schematisch die Detektion von Streulicht auf einem planaren Träger.

Figur 7 zeigt schematisch die Messung von Polymerisation auf planaren (A) und porösen (B) Trägern mittels Impedanzauswertung kapazitiver Änderungen.

Die vorliegende Erfindung wird durch die nachfolgenden Beispiele näher erläutert.

**Beispiel 1: Sandwich Elisa (vgl. Figur 2)**

Ein Maus-Antikörper (Primärantikörper, AK1), erkennt ein zu detektierendes Antigen (AG) aus dem Menschen. AG ist am Boden einer Mikrotiterplatte gebunden und wird von AK1 gebunden. Man inkubiert den Komplex (= Analyt) mit einem anti-Maus Immunglobulin (Sekundärantikörper, AK2 = Detektormolekül) der durch bekannte Methoden mit einem Biotinmolekül konjugiert ist (erhältlich von Sigma, Rockland etc.). Nach einem Waschschritt inkubiert man den entstandenen Sandwichkomplex (siehe oben) mit einem Avidin-Glukoseoxidasekonjugat (KON = Katalysatorsystem) und gibt gleichzeitig oder später eine Mischung aus Glucose (Substrat) sowie eine Lösung aus Acrylamid (Monomer) und einem der oben beschriebenen geeigneten Reduktionsmittel (Initiator) dazu (LSG1). KON bindet über Avidin/Biotin an AK2, die GOD bildet $H_2O_2$, wodurch über den Initiator ein Hydroxylradikal entsteht, das über die radikalische Kettenreaktion ein Acrylamid zu einem Polyacrylamid kondensiert. Dieser Vorgang läuft - wie oben beschrieben - unabhängig von der Neubildung weiterer Hydroxylradikale ab. Durch Zugabe von Bisacrylamid zu LSG1 kann man das Polymerisat zusätzlich quervernetzen und Porosität und Wasserquellbarkeit steuern. Nach wenigen Sekunden bis Minuten hat sich ein Polymer gebildet, das z.B. über Absorptions- oder Reflektionsmessung erfaßt werden kann.

**Beispiel 2: Detektion von Nukleinsäuren (vgl. Figur 3)**

Ein Oligonukleotid bekannter Sequenz (SS1 = Analyt) wird auf einem geeigneten Substrat durch bekannte Methoden fixiert. Aus einem Gemisch menschlicher mRNA wird über die PCR eine cDNA erstellt, wobei man in der PCR bestimmte mit einem Biotinmolekül versehene Basen anbietet, die entsprechend über die gesamte Länge der cDNA an den entsprechenden Stellen eingebaut werden. Nach der Denaturierung hybridisiert der komplementäre Einzelstrang (SS2 = Detektormolekül) mit SS1. Im Anschluß gibt man wie in Beispiel 1 beschrieben KON (= Katalysatorsystem) und LSG1 hinzu.

**Beispiel 3: Detektion von Nukleinsäuren; primär markierungsfrei (vgl. Figur 4)**

Ein Oligonukleotid bekannter Sequenz (SS1) wird auf einem geeigneten Substrat durch bekannte Methoden fixiert. Aus dem Gemisch menschlicher mRNA wird über die PCR eine cDNA erstellt, wobei einer der Primer so gewählt wird, daß nach der Hybridisierung des komplementären Einzelstrangs (SS3) beispielsweise an seinem 3'-Ende ca. 10 bis 20 weitere ungepaarte Basen entstehen (SS3*). Der eigentliche Hybridisierungsvorgang verläuft also in diesem Beispiel ohne eine mögliche Interferenz mit einem eingebauten Marker. Die ungepaarten Basen dienen als Hybridisierungsstelle eines weiteren Oligonukleotids, das mit einem Biotin konjugiert ist (SS4 = Detektormolekül). Man hybridisiert SS4 an SS3*, wobei die Bedingungen so gewählt werden, daß SS1 an SS3 gebunden bleibt. Im Anschluß gibt man wie oben beschrieben KON (= Katalysatorsystem) und LSG1 hinzu.

## Patentansprüche

1. Analysesystem, enthaltend ein Detektormolekül, welches zur spezifischen Bindung an zu bestimmende Analyten befähigt ist, und ein Katalysatorsystem, welches an das Detektormolekül gebunden vorliegt oder zur spezifischen Bindung an das Detektormolekül befähigt ist, wobei das Katalysatorsystem (i) zur katalytischen Herstellung eines Polymerisats oder (ii) zur katalytischen Erzeugung eines Polymerisationsinitiators oder einer Vorstufe davon für die Herstellung eines Polymerisats befähigt ist.

2. Analysesystem nach Anspruch 1, worin ein Detektormolekül mindestens einen Analyt-bindenden Bereich und mindestens einen Katalysatorsystem-bindenden Bereich umfaßt, wobei diese Bereiche unabhängig voneinander aus der Gruppe, bestehend aus Antigen-bindenden Antikörperstrukturen, Haptenstrukturen, Nukleinsäuresequenzen oder komplementären Sequenzen/Strukturen-bindenden Derivaten davon, Ligandensystemen und Polypeptid-affinen Systemen, ausgewählt sind.

3. Analysesystem nach Anspruch 2, wobei das Detektormolekül ein Antikörper oder eine Nukleinsäuresequenz, jeweils mit mindestens einem konjugierten Biotinmolekül, ist.

4. Analysesystem nach einem der Ansprüche 1 bis 3, wobei das Katalysatorsystem mindestens einen Detektormolekül-bindenden Bereich umfaßt, wobei der Bereich aus der Gruppe, bestehend aus Antigen-bindenden Antikörperstrukturen, Haptenstrukturen, Nukleinsäuresequenzen oder komplementären Sequenzen/Strukturen-bindenden Derivaten davon, Ligandensystemen und Polypeptid-affinen Systemen, ausgewählt ist.

5. Analysesystem nach Anspruch 4, wobei der Detektormolekül-bindende Bereich Avidin umfaßt.

6. Analysesystem nach Anspruch 1, worin ein Detektormolekül mindestens einen Analyt-bindenden Bereich und an das Detektormolekül kovalent gebunden mindestens ein Katalysatorsystem umfaßt, wobei der Analyt-bindende Bereich aus der Gruppe, bestehend aus Antigen-bindenden Antikörperstrukturen, Haptenstrukturen, Nukleinsäuresequenzen oder komplementären Sequenzen/Strukturen-bindenden Derivaten davon, Ligandensystemen und Polypeptid-affinen Systemen, ausgewählt ist.

7. Analysesystem nach einem der Ansprüche 1 bis 6, wobei das Katalysatorsystem mindestens einen Bereich zur katalytischen Erzeugung eines Polymerisationsinitiators oder einer Vorstufe davon umfaßt, wobei der Bereich mindestens ein Enzym aus der Gruppe der Oxidasen umfaßt.

8. Analysesystem nach Anspruch 7, wobei die Oxidasen aus der Gruppe, bestehend aus Lactatoxidase, Galactoseoxidase, L-2-Hydroxysäureoxidase, Glucoseoxidase, Glycolatoxidase, Hexoseoxidase, L-Gulonolactonoxidase, L-Sorboseoxidase, Pyridoxol-4-oxidase und Alkoholoxidase, ausgewählt sind.

9. Analysesystem nach einem der Ansprüche 1 bis 8, wobei der Polymerisationsinitiator ein radikalischer oder ein ionischer Polymerisationsinitiator ist.

10. Analysesystem nach einem der Ansprüche 1 bis 9, wobei das durch Lösungs-, Emulsions- oder Suspensionspolymerisation herzustellende Polymerisat aus polymerisierbaren Verbindungen aufgebaut ist.

11. Analysesystem nach Anspruch 10, wobei die polymerisierbaren Verbindungen Monomere und/oder Oligomere umfassen.

**12.** Analysesystem nach Anspruch 10 oder 11, wobei die polymerisierbaren Verbindungen aus der Gruppe, bestehend aus $\alpha,\beta$-ungesättigten Mono- oder Dicarbonsäuren, $\alpha,\beta$-ungesättigten Carbonsäureamiden, hydroxylgruppenhaltigen Monomeren mit mindestens einer polymerisierbaren ethylenisch ungesättigten Gruppe und aminogruppenhaltigen Monomeren mit mindestens einer polymerisierbaren ethylenisch ungesättigten Gruppe, ausgewählt sind.

**13.** Analysesystem nach einem der Ansprüche 10 bis 12, wobei das Polymerisat ein Homo- oder Copolymerisat ist.

**14.** Analysesystem nach einem der Ansprüche 1 bis 13, wobei der zu bestimmende Analyt eine niedermolekulare organische Verbindung oder ein biologisches Material ist.

**15.** Analysesystem nach Anspruch 14, wobei das biologische Material aus der Gruppe, bestehend aus Polypeptiden oder Fragmenten und Derivaten davon, einzel- oder doppelsträngig vorliegenden Nukleinsäuren oder Fragmenten und Derivaten davon, Lipiden, Hormonen, Stoffwechselprodukten, Zuckerverbindungen, eukaryontischen und prokaryontischen Zellen oder Zellbestandteilen davon, Antigen-Antikörper-Komplexen und Rezeptor-Liganden, ausgewählt ist.

**16.** Kit, enthaltend das Analysesystem nach einem der Ansprüche 1 bis 15.

**17.** Verfahren zum Nachweis von auf einem Träger immobilisierten Analyten, umfassend die Schritte:

(a) Inkontaktbringen des immobilisierten Analyten mit dem in einem der Ansprüche 1 bis 9 definierten Analysesystem unter Ausbildung eines Analyt-Analysesystem-Komplexes,

(b) Starten einer Polymerisation

(i) nach Zugabe der in den Ansprüchen 11 und 12 definierten polymerisierbaren Verbindungen oder
(ii) nach Zugabe eines Substrats zur katalytischen Erzeugung eines Polymerisationsinitiators oder einer Vorstufe davon und der polymerisierbaren Verbindungen

unter Herstellung eines in einem der Ansprüche 10 bis 13 definierten Polymerisats und

(c) Auswerten der Polymerisation unter Bestimmung des Polymerisats.

**18.** Verfahren nach Anspruch 17, wobei das in Schritt (a) verwendete Detektormolekül ein Antikörper oder eine Nukleinsäure, jeweils mit mindestens einem konjugierten Biotinmolekül, ist und das in Schritt (a) verwendete Katalysatorsystem Avidin umfaßt.

**19.** Verfahren nach Anspruch 17 oder 18, wobei das Inkontaktbringen von Schritt (a) eine Antigen-Antikörper-Reaktion oder eine Hybridisierung ist.

**20.** Verfahren nach einem der Ansprüche 17 bis 19, wobei das Katalysatorsystem das Enzym Glucoseoxidase umfaßt und die in Schritt (b) erzeugte Vorstufe des Polymerisationsinitiators $H_2O_2$ ist.

**21.** Verfahren nach einem der Ansprüche 17 bis 20, wobei die in Schritt (c) durchgeführte Bestimmung des Polymerisats optisch, elektrisch und/oder gravimetrisch erfolgt.

**22.** Verfahren nach einem der Ansprüche 17 bis 21, wobei der Träger aus einem, gegebenenfalls porösen Halbleitermaterial aufgebaut ist.

**23.** Verfahren nach Anspruch 22, wobei das Halbleitermaterial aus der Gruppe, bestehend aus Silizium, Siliziumoxid und Silizium mit mindestens einer oder mehrerer Schichten, ausgewählt aus Siliziumoxid, Siliziumnitrid, Germanium und Galliumarsenid, ausgewählt ist.

**Fig. 1**

(A)

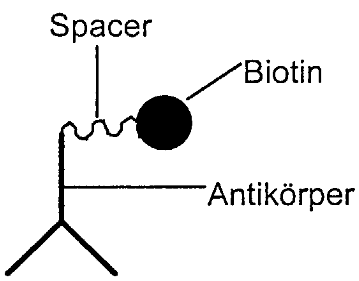
Spacer
Biotin
Antikörper

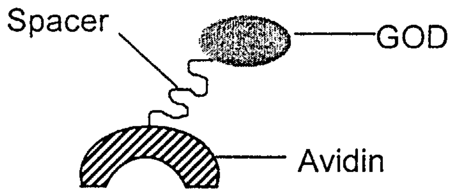
Spacer
GOD
Avidin

(B)

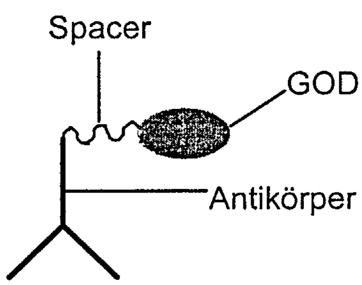
Spacer
GOD
Antikörper

Fig. 2

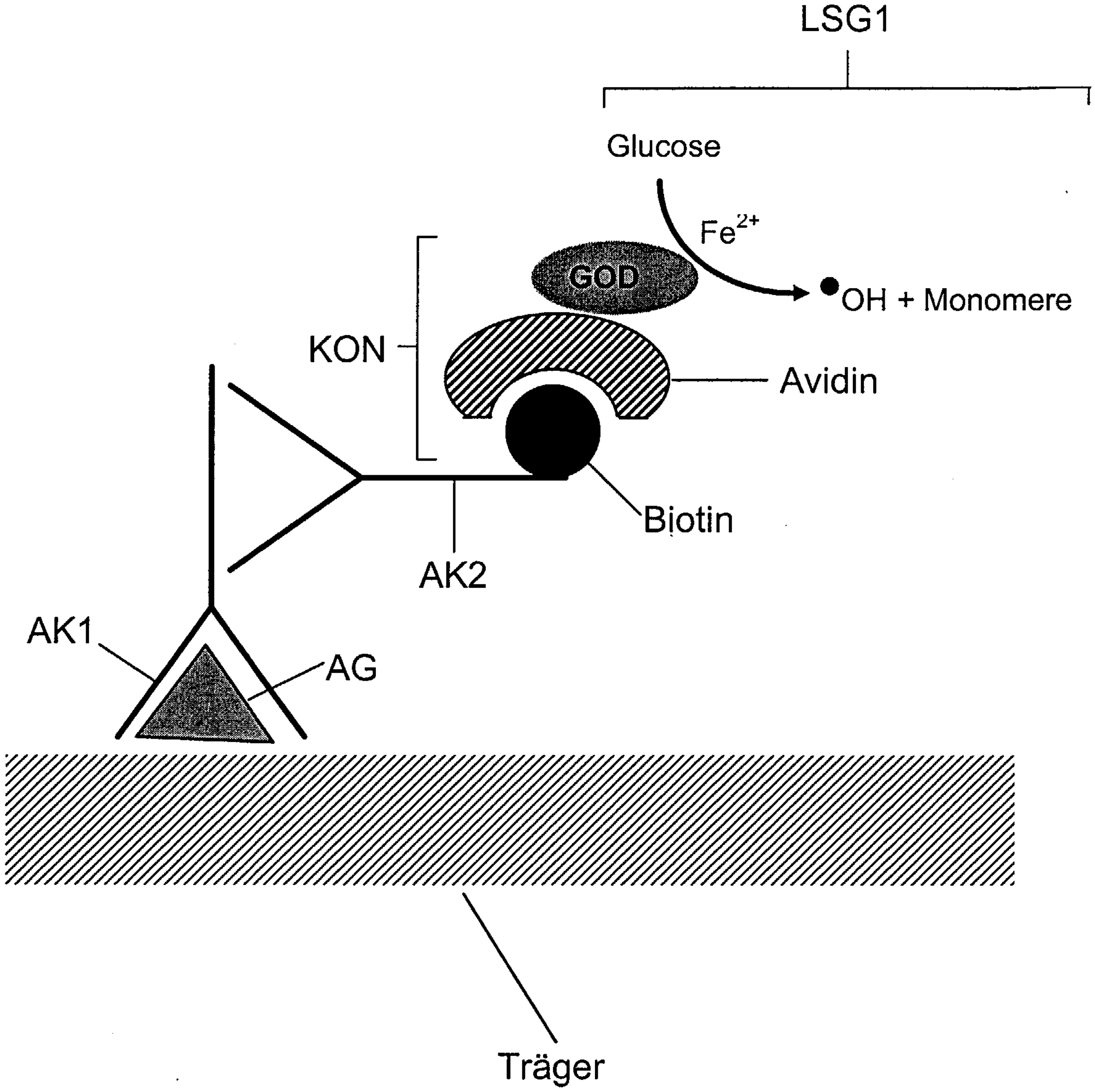
LSG1
Glucose
Fe2+
GOD
•OH + Monomere
KON
Avidin
Biotin
AK2
AK1
AG
Träger

Fig. 3

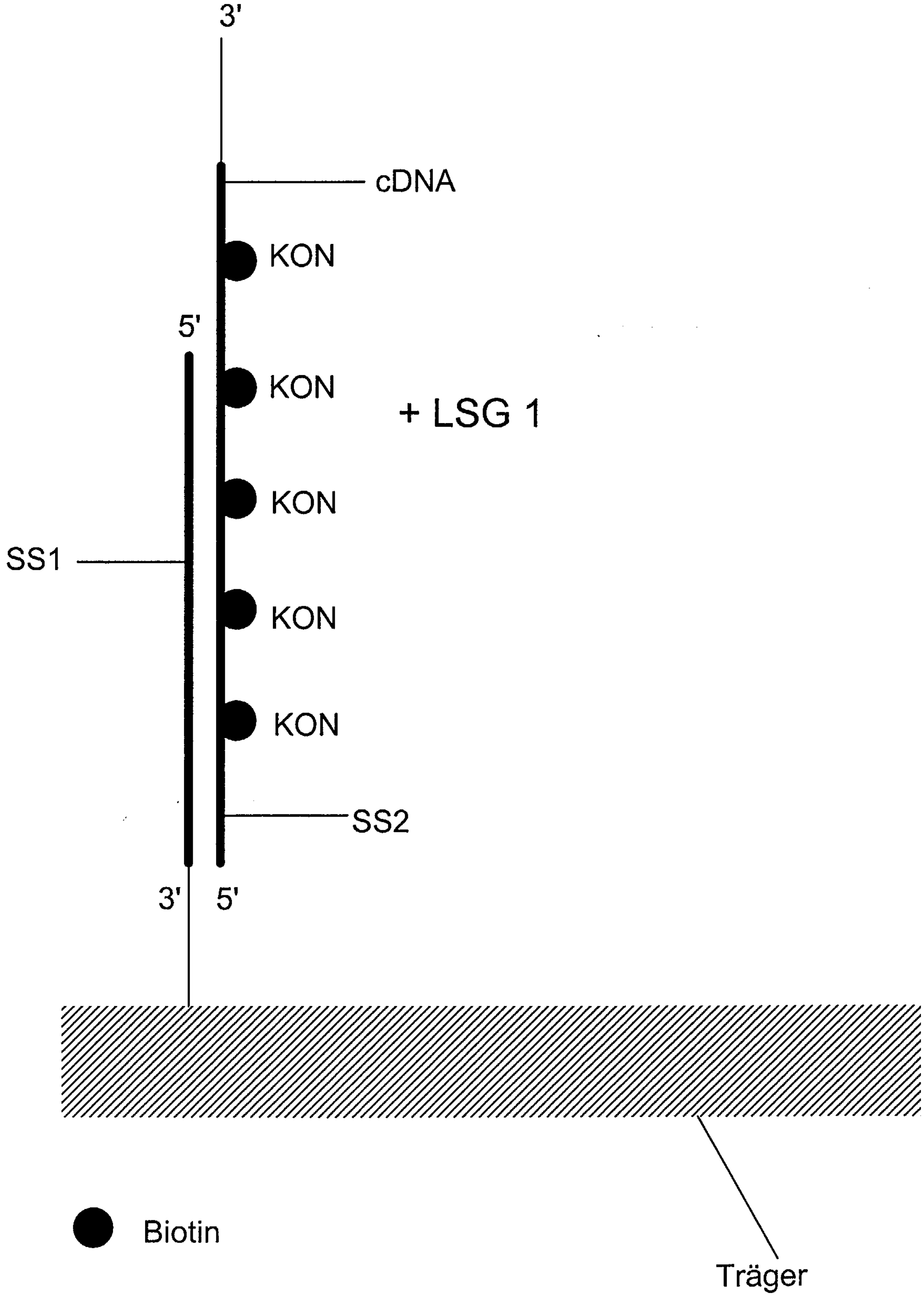

Fig. 4

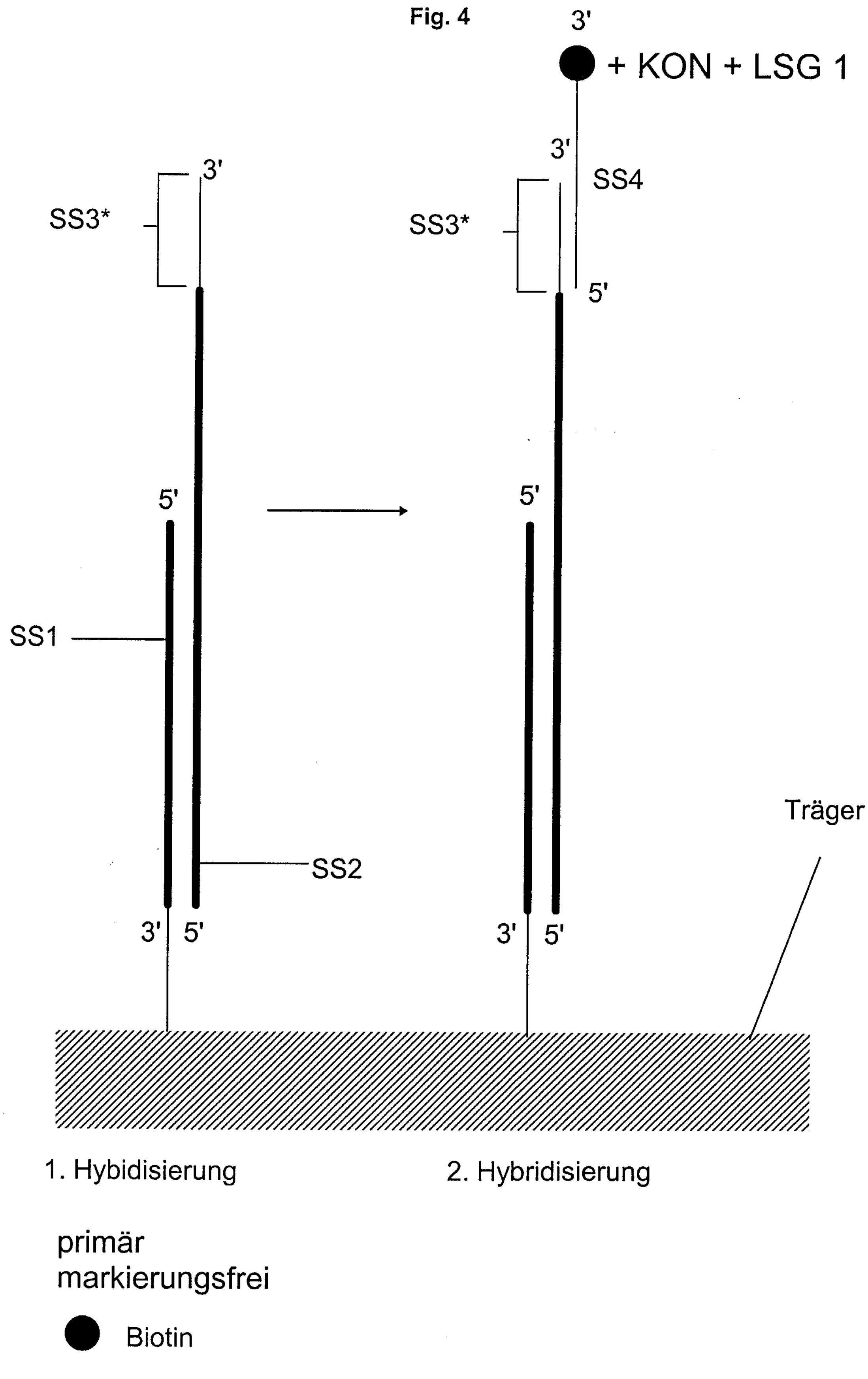
3'
+ KON + LSG 1
3'
SS3*
3'
SS4
SS3*
5'
5'
5'
SS1
SS2
3'
5'
3'
5'
Träger
1. Hybidisierung
2. Hybridisierung
primär
markierungsfrei
Biotin

Fig. 5A

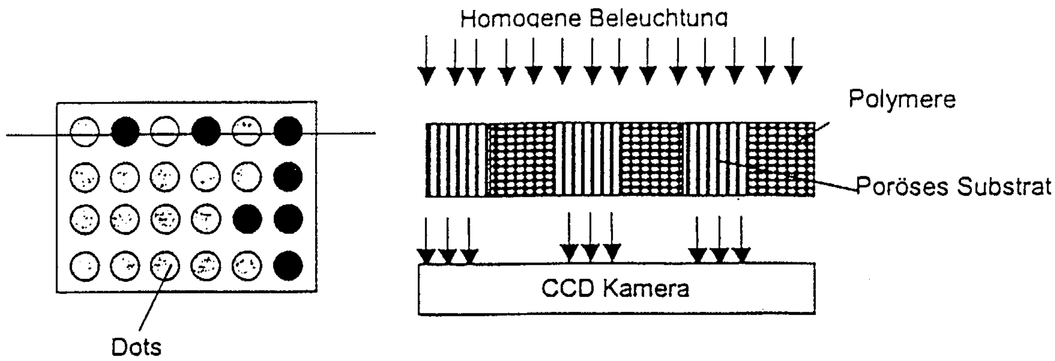


Fig. 5B

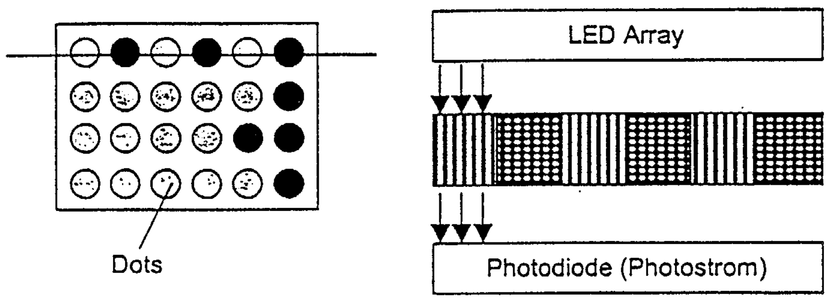


Fig. 6

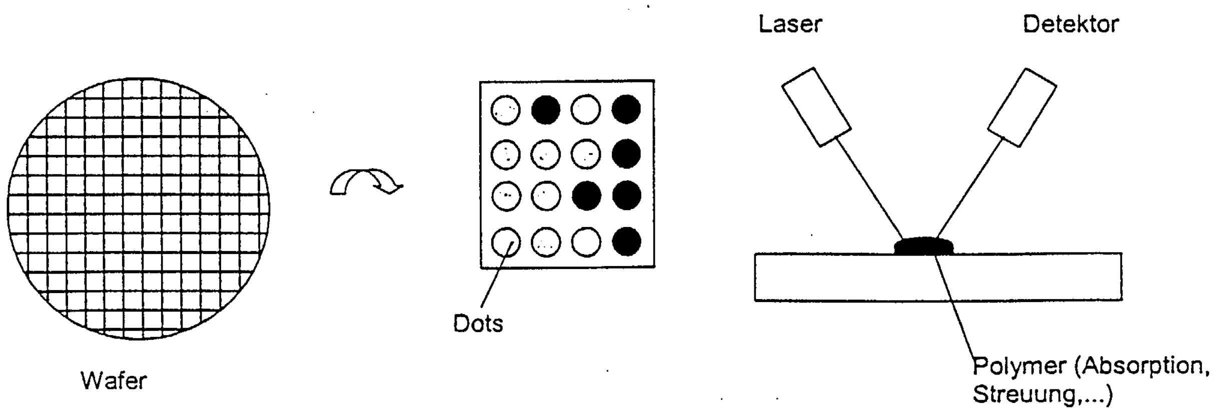

Fig. 7A

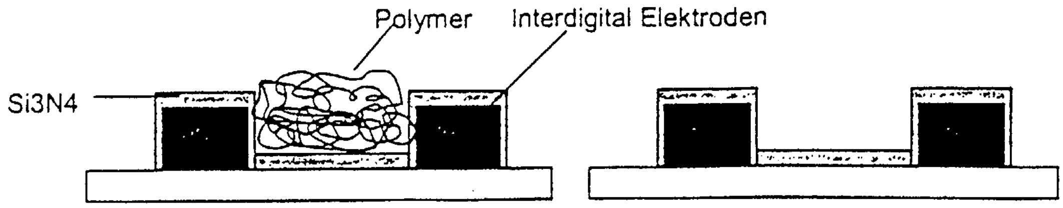
Polymer
Interdigital Elektroden
Si3N4

Fig. 7B

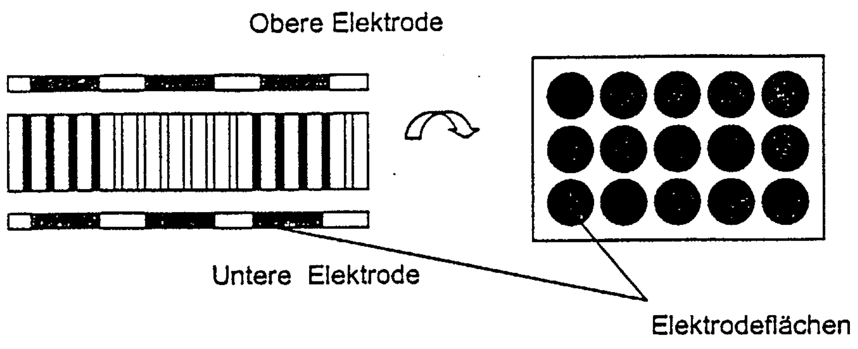
Obere Elektrode
Untere Elektrode
Elektrodeflächen

Europäisches Patentamt

# EUROPÄISCHER RECHERCHENBERICHT

Nummer der Anmeldung
EP 03 00 4841

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X<br><br><br>Y | US 5 035 997 A (DAVIS BARUCH J ET AL) 30. Juli 1991 (1991-07-30)<br>* das ganze Dokument * | 1-21<br><br><br>17,21-23 | G01N33/58<br>C12Q1/26<br>G01N33/543<br>G01N33/552 |
| X | WO 89 06362 A (UNIV WALES MEDICINE) 13. Juli 1989 (1989-07-13)<br>* das ganze Dokument * | 1-6,9-19 | |
| X<br><br>Y | KRICKA L J: "SELECTED STRATEGIES FOR IMPROVING SENITIVITY AND RELIABILITY OF IMMUNOASSAYS" CLINICAL CHEMISTRY, AMERICAN ASSOCIATION FOR CLINICAL CHEMISTRY. WINSTON, US, Bd. 40, Nr. 3, 1994, Seiten 347-357, XP001074218 ISSN: 0009-9147<br>* Seite 352, linke Spalte, Zeile 32 - rechte Spalte, Zeile 28 * | 1-4,6, 10,11, 13-19,21<br><br>17,21-23 | |
| | | | RECHERCHIERTE SACHGEBIETE (Int.Cl.7) |
| Y | DATABASE COMPENDEX 'Online! ENGINEERING INFORMATION, INC., NEW YORK, NY, US; GU JI-DONG ET AL: "Microbial degradation of polymeric coatings measured by electrochemical impedance spectroscopy" Database accession no. EIX99034472052 XP002246100<br>* Zusammenfassung *<br>& BIODEGRADATION;BIODEGRADATION 1998 KLUWER ACADEMIC PUBLISHERS, DORDRECHT, NETHERLANDS, Bd. 9, Nr. 1, 1998, Seiten 39-45, | 17,21 | G01N<br>C12Q |

-/--

Der vorliegende Recherchenbericht wurde für alle Patentansprüche erstellt

| Recherchenort | Abschlußdatum der Recherche | Prüfer |
|---|---|---|
| DEN HAAG | 1. Juli 2003 | Tuynman, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

Europäisches Patentamt

# EUROPÄISCHER RECHERCHENBERICHT

Nummer der Anmeldung
EP 03 00 4841

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | DATABASE COMPENDEX 'Online!<br>ENGINEERING INFORMATION, INC., NEW YORK, NY, US;<br>ANWARUDDIN Q ET AL: "VINYL POLYMERIZATION PHOTOSENSITIZED BY ANTHRAQUINONE SULFONATES IN AQUEOUS SOLUTIONS"<br>Database accession no. EIX70010012587<br>XP002246101<br>* Zusammenfassung *<br>& J POLYMER SCIENCE-POLYMER CHEM MAY 1969, Bd. 7, Nr. 5 part A-1, Mai 1969 (1969-05), Seiten 1315-30,<br>--- | 17,21 | |
| Y | PATENT ABSTRACTS OF JAPAN<br>vol. 2000, no. 23,<br>10. Februar 2001 (2001-02-10)<br>& JP 2001 149059 A (OLYMPUS OPTICAL CO LTD), 5. Juni 2001 (2001-06-05)<br>* Zusammenfassung *<br>--- | 17,21-23 | |
| A | WO 99 60096 A (JOO HYUN ;LIN ZHANGLIN (US); ARNOLD FRANCES H (US); CALIFORNIA INS) 25. November 1999 (1999-11-25)<br>* das ganze Dokument *<br>----- | 1-23 | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| DEN HAAG | 1. Juli 2003 | Tuynman, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 03 00 4841

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-07-2003

| Im Recherchenbericht angeführtes Patentdokument | Datum der Veröffentlichung | Mitglied(er) der Patentfamilie | Datum der Veröffentlichung |
|---|---|---|---|
| US 5035997 A | 30-07-1991 | EP 0383124 A2 | 22-08-1990 |
| | | JP 2259567 A | 22-10-1990 |
| WO 8906362 A | 13-07-1989 | AU 2938789 A | 01-08-1989 |
| | | EP 0440611 A1 | 14-08-1991 |
| | | WO 8906362 A1 | 13-07-1989 |
| JP 2001149059 A | 05-06-2001 | KEINE | |
| WO 9960096 A | 25-11-1999 | AU 4200999 A | 06-12-1999 |
| | | CA 2328412 A1 | 25-11-1999 |
| | | EP 1216308 A2 | 26-06-2002 |
| | | WO 9960096 A2 | 25-11-1999 |
| | | AU 5134599 A | 21-02-2000 |
| | | CA 2331777 A1 | 10-02-2000 |
| | | EP 1100891 A2 | 23-05-2001 |
| | | JP 2003503005 T | 28-01-2003 |
| | | WO 0006718 A2 | 10-02-2000 |

EPO FORM P0461

Für nähere Einzelheiten zu diesem Anhang : siehe Amtsblatt des Europäischen Patentamts, Nr.12/82